Europäisches Patentamt

European Patent Office

Office européen des brevets

(11) Veröffentlichungsnummer: **0 278 360**

**A2**

## (12) EUROPÄISCHE PATENTANMELDUNG

(21) Anmeldenummer: **88101440.1**

(22) Anmeldetag: **02.02.88**

(51) Int. Cl.⁴: **A61L 2/18**

(30) Priorität: **12.02.87 DE 8702105 U**
**25.04.87 DE 8706008 U**

(43) Veröffentlichungstag der Anmeldung:
**17.08.88 Patentblatt 88/33**

(84) Benannte Vertragsstaaten:
**CH FR GB IT LI NL SE**

(71) Anmelder: **Nielsen, Ernst Peter Friedrich**
**Berliner Strasse 24**
**D-6090 Rüsselsheim(DE)**

(72) Erfinder: **Nielsen, Ernst Peter Friedrich**
**Berliner Strasse 24**
**D-6090 Rüsselsheim(DE)**

(54) **Vorrichtung zur Reinigung von medizinischen Handinstrumenten sowie Handgeräten für die Haar- Haut- bzw. Körperpflege.**

(57) Angesichts der heute erhöhten Gefahren für Ärzte und Personal durch verbreitete alte und neue Virusinfektionen, z.B. Hepatitis-B und Aids, erfolgt die Vorreinigung medizinischer Handinstrumente mittels mechanischer Reinigungsmittel, z.B. Bürsten (38, 39), Lamellen oder Kunststoffkissen, die sich in einem gegebenenfalls geschlossenen Reinigungsbehälter (31) oder einer Reinigungsschale (3o) unter dem Flüssigkeitsspiegel einer Desinfektionslösung befinden.

Es werden vorteilhafte Ausführungen vorgeschlagen, die ein Verspritzen kotaminierter Teilchen in die Umgebung verhindern. In diesem Zusammenhang wird auch eine zusätzliche Desinfektionsschale (41) am Arbeitsplatz vorgeschlagen, die als Soforthilfe nach Verletzungen beim Hantieren am Arbeitsplatz dient.

Die Erfindung kann auch in nichtärztlichen Praxen, z.B. in Friseur-Manikür-Pedikür-Kosmetik und Tätovierbetrieben angewendet werden, um deren Personal und Kunden vor Infektionen zu schützen.

FIG.3

## VORRICHTUNG ZUR REINIGUNG VON MEDIZINISCHEN HANDINSTRUMENTEN SOWIE HANDGERÄTEN FÜR DIE HAAR-HAUT BZW. KÖRPERBEHANDLUNG

Die Erfindung betrifft vornehmlich eine Vorrichtung von medizinischen Handinstrumenten innerhalb von Arztpraxen, medizinischen und biologischen Laboratorien, sowie Operationssälen.

Nach dem bestimmungsgemäßen Gebrauch von medizinischen Handinstrumenten ist es üblich, diese vor einer abschließenden Sterilisation im Autoklaven einer Vorreinigung zu unterziehen. Dies erfolgt im allgemeinen in der Weise, daß die über eine gewisse Zeitspanne gesammelten Instrumente über einem Waschbecken mittels mechanischer Reinigungsmittel, z.B. Bürsten oder dgl. , unter fließendem Wasser vorgereinigt werden. Bei dieser Reinigung werden an den Instrumenten anhaftende oft schon verklebte oder verkrustete Gewebeteile, Blut, Speichel usw., mechanisch entfernt. Danach gelangen die Instrumente zu weiteren Behandlung in eine Desinfektionslösung.

Während der vorstehend beschriebenen Grobreinigung der Instrumente sind das Personal und die Ärzte erhöhten Infektionsgefahren ausgesetzt. Das mechanische Abstreifen, bzw. Bürsten schließt - auch bei umsichtiger Handhabung - keineswegs aus, daß Partikel der an den Instrumenten anhaftenden kontaminierten Stoffe verspritzt werden und schon bei winzigen Verletzungen der handhabenden Personen in deren Blutbahn gelangen können. In zahnärztlichen Praxen beispielsweise kommt es bei der Behandlung eines Patienten vor, daß sich der Arzt an den Händen verletzt. Das Tragen von Gummihandschuhen stellt keinen ausreichenden Schutz dar, da das dünne Material der Handschuhe bei der Verletzung perforiert und damit die Gefahr einer unmittelbaren Infiltration infektiöser Stoffe entsteht.

Diese Gefahren sind heute, angesichts sich verbreitender alter und neuer Virusinfektionen, z.B. Hepatitis-B und Aids, besonders groß.

Nach der Erfindung kann die Vorrichtung fernerzur Reinigung von Handgeräten für die Haar-Haut-bzw. Körperbehandlung dienen, wie sie z.B. in Friseur-Pedikür-Manikür-und Kosmetikbetrieben in Gebrauch sind.

Im wesentlichen bestehen diese Handgeräte aus Bürsten, Scheren, Kämmen, Pinzetten, Messern, Pinseln und dgl. mehr, sowie auch aus speziellen Geräten für das Tätovieren der Haut. In zunehmendem Maße finden in den vorgenannten Betrieben auch medizinische Handinstrumente Eingang.

Trotz geschicktester Handhabung 11 dieser Handgeräte, sind Verletzungen der Kunden, z.B. auf der Kopfhaut beim Haareschneiden, beim Rasieren, bei der Entfernung von Mitessern, beim Nägelschneiden und dgl. mehr nicht auszuschließend. Im allgemeinen werden beispielsweise Kämme, Scheren und Messer in Friseurbetrieben keiner besonderen mechanischen Desinfektions-Reinigung unterzogen. Häufig kann beobachtet werden, daß Handgeräte lediglich abgsprüht oder mit desinfizierenden Tüchern abgewischt werden, um verklebte etwaige kontaminierte Stoffe, wie Hautgrind, Schorf, Blut, Eiter usw. zu entfernen. In all diesen Fällen kann beim Gebrauch der Handgeräte eine Infizierung der Kunden und Bedienenden nicht ausgeschlossen werden.

Die Erfindung hat sich zur Aufgabe gemacht, den geschilderten Gefahren praxisgerecht Einhalt zu gebieten.

Nach der Erfindung wird diese Aufgabe dadurch gelöst, daß zur Reinigung verwendete mechanische Reinigungsmittel, z.B. Bürsten, Lamellen oder Schaumstoffkissen, innerhalb eines Gefäßes unter dem Flüssigkeitsspiegel angeordnet sind. Hierdurch wird unbeabsichtigtes Verspritzen kontaminierter Stoffe z.B. ins Gesicht, an die Hände, in den angrenzenden Arbeitsbereich oder gar in die Raumluft, vermieden. Die Reinigungsmittel sind Einwegartikel. Sie werden daher leicht und rasch auswechselbar, z.B. mittels Steckverbindungen innerhalb des Gefäßes befestigt.

Es wird ferner vorgeschlagen, das Gefäß mit einem Deckel zu versehen der einen länglichen Schlitz aufweist, durch den die medizinischen Handinstrumente in den Behälter zur Berührung mit den Reinigungsmitteln eingeführt werden können. Hierdurch wird zusätzlich zu dem Hauptmerkmal der Erfindung ein Verspritzen kontaminierter Stoffe weitgehend ausgeschaltet.

Mindestens der Deckel sollte aus durchsichtigem Material, z.B. Glas, bestehen. Der Bedienende sieht auf diese Weise, wo und wie er die Instrumente bewegt und kann den Fortschritt der Vorreinigung kontrollieren.

Um eine intensive Säuberung der Instrumente zu erreichen, sind zwei Reinigungsmittel spiegelbildlich und horizontal so zueinander angeordnet, daß sie sich berühren. Zur weiteren Intensivierung der Reinigung kann am Boden des Gefäßes ein drittes Reinigungsmittel vorgesehen sein.

Die Reinigungsmittel können nach einem weiteren Merkmal der Erfindung an Wänden des Behälters oder an Teilen des Deckels befestigt sein. Im letzteren Fall ist der Deckel als ein nach unten offenes Gefäß ausgebildet, wodurch insbesondere ein Überschwappen von Flüssigkeit bei zu rascher Hin-und Herbewegung der Instrumente während der Reinigung vermieden wird.

Eine vereinfachte Ausführung des Erfindungsgedankens besteht darin, ein oder mehrere Reinigungsmittel mit geeigneten Steckverbindungen im Siebeinsatz einer an sich bekannten Desinfektionsschale zu befestigen. In diesem Fall können die im Siebeinsatz ohnehin vorhandenen Löcher gleichzeitig zur Befestigung des Reinigungsmittels dienen.

Um mit Sicherheit Infektionen weitgehend auszuschalten, wird erfindungsgemäß als Soforthilfe im Fall einer Verletzung am Behandlungs- bzw. Arbeitsplatz eine mit einer vorgenannten Desinfektionsschale kombinierte kleinere Schale vorgeschlagen, die ein mit einer speziellen virus- und keimtötenden Lösung getränktes Kissen enthält. Durch diese Anordnung wird die Möglichkeit geboten, daß beispielsweise der Behandelnde im Fall einer Verletzung an den Händen durch unverzügliches Handeln die blutmäßige Übertragung von Viren und Keimen inhibieren kann.

Nachfolgend ist die Erfindung an hand von Ausführungsbeispielen dargestellt und näher beschrieben.

Fig. 1 zeigt ein erstes Ausführungsbeispiel der Vorrichtung in räumlicher Darstellung.

Fig. 2 ist ein Schnitt entlang der Linien 2 - 2 in Fig. 1.

Fig. 3 zeigt ein zweites Ausführungsbeispiel der Vorrichtung in räumlicher Darstellung.

Fig. 4 ist ein Schnitt entlang der Linien 4 - 4 in Fig. 3.

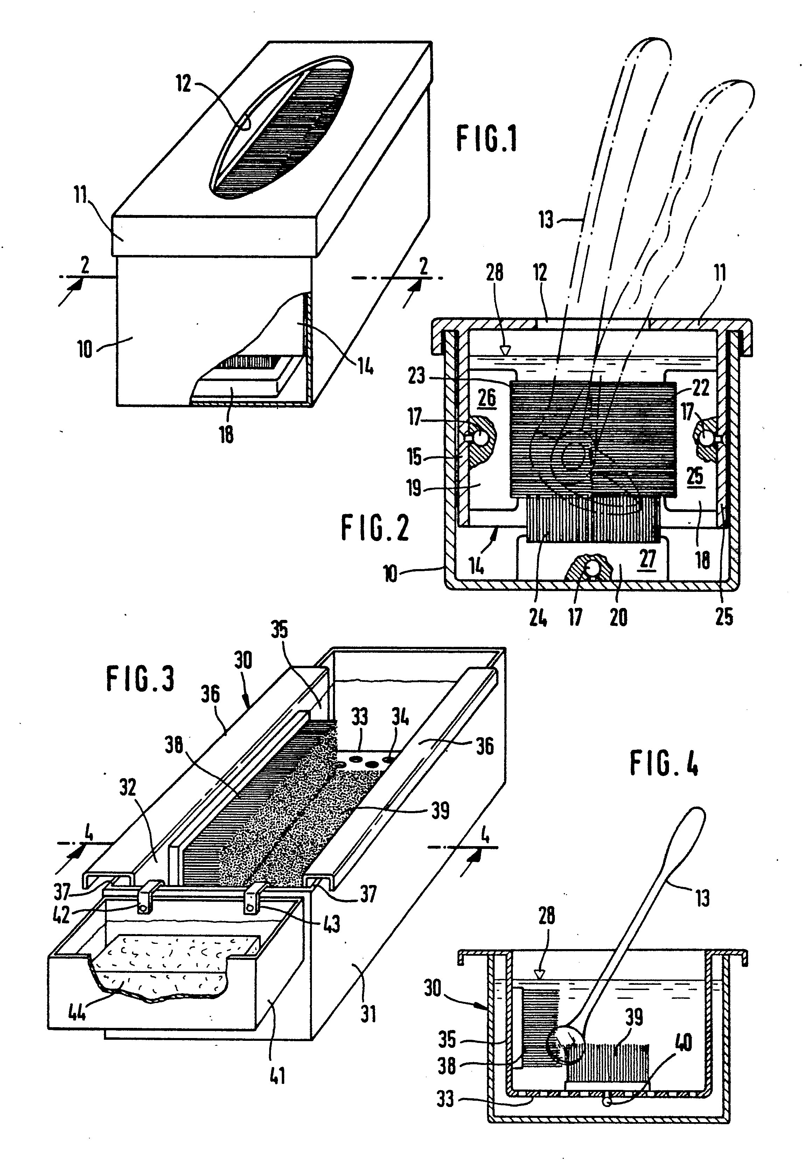

Die in den Figuren 1 und 2 dargestellte Vorrichtung besteht aus einem Behälter 1o und einem daraufliegenden Deckel 11. Der Behälter 1o hat einen rechteckigen Boden und entsprechende Seitenwände. Als Größe des Behälters 1o wird etwa 3o x 15 x 15 cm L x B x H, vorgeschlagen. Behälter 1o und Deckel 11 sind zweckmäßigerweise aus einem durchsichtigen Material, am besten Glas, gefertigt. Im Deckel 11 befindet sich ein länglicher, ovaler Schlitz 12 zur Einführung der mechanischen Handinstrumente, wie in Fig.2 mit 13 angedeutet. Der Deckel 11 ist als ein nach unten offenes Gefäß 14 mit Wänden 15 ausgebildet. Die Außenseiten der Wände 15 befinden sich allseitig möglichst nahe an den Innenwänden des Behälters 1o um ein Überschwappen der Flüssigkeit zu vermeiden. An den Innenwänden des nach unten offenen Gefäßes 14 sind die Reinigungsmittel mit Steckverbindungen 17 bekannter Baurt befestigt.

Im Ausführungsbeispiel nach den Fig. 1 und 2 sind es drei Bürsten 18, 19, 2o von denen zwei 18 und 19 spiegelbildlich und horizontal zueinander angeordnet sind und eine dritte Bürste 2o am Boden des Behälters 1o mit nach oben weisenden Borsten befestigt ist. Die Bürsten 18, 19, 2o haben vorzugsweise gleiche Größe. Ihre Borsten 22, 23, 24 sind in ihren Sockeln 25, 26, 27 eingegossen. Die Bürsten sind so angeordnet, daß sich ihre Borsten berühren. Alle Teile der Bürsten sind aus säurefestem Material bekannter Zusammensetzung gefertigt. Mit 28 ist der Flüssigkeitsspiegel einer an sich bekannten Desinfektionslösung bezeichnet.

Das Ausführungsbeispiel gemäß den Figuren 3 und 4 geht von einer üblichen Desinfektionsschale 3o aus. Sie besteht aus einem Behälter 31 mit etwa ähnlichen Abmessungen wie der Behälter 1o der Figur 1 und einem Siebeinsatz 32. Das Siebblech 33 mit einer Vielzahl von Löchern 34 ist an seinen Längsseiten zur Bildung zweier Schenkel 35 nach oben abgewinkelt. Die beiden Schenkel 35 besitzen an ihren oberen Enden Abbördelungen 36, die auf den Rändern 37 der Infektionsschale 3o ruhen. Die Reinigungsmittel sind auf dem Siebblech 33 und/oder an den Schenkeln 35 angeordnet. Im Ausführungsbeispiel sind nur an einem Schenkel 35 und dem Siebblech 33 Bürsten 38, 39 mittels Steckverbindung 4o befestigt. Selbstverständlich können auch an beiden Schenkeln 35 Bürsten vorgesehen sein.

Als Soforthilfe bei einer Verletzung am Behandlungsplatz ist die Desinfektionsschale 3o mit einer kleineren Schale 41 lösbar kombiniert. Hierzu dienen zwei Aufhängungen 42, 43 die den Rand 37 der Desinfektionsschale umgreifen. In der kleineren Schale 41 befindet sich ein Kunststoffkissen 44, das mit einer speziellen virus-und keimtötenden Lösung getränkt ist. Die hantierenden Personen haben so die Möglichkeit der sofortigen Desinfektion bei Verletzung z. B. ihrer Hände. Die umittelbare Nachbarschaft der Desinfektionsschale 3o mit der kleineren Schale 41 und deren Aufstellung neben dem Arbeitsplatz des Behandlers ist von besonderer Bedeutung.

Selbstverständlich kann die kleinere Schale 41 auch mit dem Behälter 1o der in Figur 1 dargestellten Vorrichtung kombiniert werden, gegebenenfalls auch mit dieser aus einem Stück bestehen.

Die in der Zeichnung dargestellten Vorrichtungen sind vornehmlich auf medizinische Anwendungsbereiche ausgerichtet, können jedoch mit kleinen Änderungen ohne erfinderischen Aufwand auch für die Verwendung in nichtmedizischen Bereichen umgestaltet werden. Solche Änderungen betreffen beispielsweise die Größe, Anordnung und Gestaltung der Reinigungsmittel.

## Ansprüche

1. Vorrichtung zur Reinigung von medizinischen Handinstrumenten sowie Handgeräten für die Haar-Haut-bzw. Körperbehandlung mittels mechanischer Reinigungsmittel, beispielsweise Bürsten, Lamellen oder Schaumstoffkissen in Wasser oder einer Desinfektionslösung,
dadurch gekennzeichnet, daß vorzugsweise mehrere mechanische Reinigungsmittel (25, 26, 27, 38, 39) innerhalb einer Behälters (1o, 3o) unter dem Flüssigkeitsspiegel (28) angeordnet sind.

2. Vorrichtung nach Anspruch 1,
dadurch gekennzeichnet, daß die Reinigungsmittel (25, 26, 27, 38, 39) leicht und rasch auswechselbar, z.B. mittels Steckverbindungen (17, 4o) befestigt sind (Figuren 2 und 4).

3. Vorrichtung nach den Ansprüchen 1 und 2,
dadurch gekennzeichnet, daß im Deckel (11) des Behälters (1o) ein länglicher Schlitz (12) vorgesehen ist, durch den die medizinischen Handinstrumente (13), bzw. Handgeräte in den Behälter (1o) zur Berührung mit den Reinigungsmitteln (25, 26, 27, 38, 39) eingeführt werden können (Figuren 1 und 2 ).

4. Vorrichtung nach den Ansprüchen 1 bis 3,
dadurch gekennzeichnet, daß zwei Reinigungsmittel (25, 26) spiegelbildlich und horizontal zueinander so angeordnet sind, daß sie sich berühren (Figur 2).

5. Vorrichtung nach den Ansprüchen 1 bis 4,
dadurch gekennzeichnet, daß unterhalb der spiegelbildlich und horizontal zueinander angeordneten Reinigungsmittel (25, 26) ein nach oben gerichtetes weiteres Reinigungsmittel (27) angeordnet ist und die vorgenannten Reinigungsmittel (25, 26) berührt (Figur 2).

6. Vorrichtung nach den Ansprüchen 1 bis 5,
dadurch gekennzeichnet, daß die speigelbildlich und horizontal zueinander angeordneten Reinigungsmittel (25, 26) an Wänden des Behälters (1o) oder an Wänden (15) des Deckels (11) befestigt sind (Figur 2).

7. Vorrichtung nach den Ansprüchen 1 bis 6,
dadurch gekennzeichnet, daß das nach oben gerichtete Reinigungsmittel (27) am Boden des Behälters (1o) befestigt ist (Figur 2).

8. Vorrichtung nach den Ansprüchen 1 bis 7,
dadurch gekennzeichnet, daß wenigstens der Deckel (11) aus durchsichtigem Material besteht (Figur 1).

9. Vorrichtung nach den Ansprüchen 1 bis 8,
dadurch gekennzeichnet, daß der Deckel (11) des Behälters (1o) ein nach unten offenes Gefäß mit Wänden (15) darstellt, deren Außenseiten sich allseitig möglichst nahe an den Innenwänden des Behälters (1o) befinden und an den Innenseiten der Wände (15) die spiegelbildlich und horizontal angeordneten Reinigungsmittel (25, 26) befestigt sind (Figur 2).

1o. Vorrichtung nach einem oder mehreren der vorangegangenen Ansprüche, dadurch gekennzeichnet, daß in dem Behälter (1o, 3o) ein Siebeinsatz (32) für daran zu befestigende Reinigungsmittel (38), z.B. Bürsten, angeordnet ist. (Figur 3 und 4).

11. Vorrichtung nach einem oder mehreren der vorangegangen Ansprüche, dadurch gekennzeichnet, daß mit dem Behälter (1o, 3o) eine kleinere Schale (41) zur Aufnahme eines mit einer Desinfektionslösung getränkten Kissens (44), z.B. durch Aufhängungen 42, 43) verbunden ist.

FIG.1

FIG.2

FIG.3

FIG.4